Europäisches Patentamt

European Patent Office

Office européen des brevets

(11) Publication number: 0 244 058 B1

# EUROPEAN PATENT SPECIFICATION

(45) Date of publication of patent specification: 23.09.92    (51) Int. Cl.⁵: **A61B 17/22**, A61B 17/32

(21) Application number: 87301542.4

(22) Date of filing: 23.02.87

(54) **Motor drive unit for use with an atherectomy catheter device.**

(30) Priority: 28.02.86 US 834743

(43) Date of publication of application:
04.11.87 Bulletin 87/45

(45) Publication of the grant of the patent:
23.09.92 Bulletin 92/39

(84) Designated Contracting States:
BE CH DE FR GB IT LI NL

(56) References cited:
EP-A- 0 163 502
US-A- 4 461 305

(73) Proprietor: DEVICES FOR VASCULAR INTER-
VENTION, INC.
595 Penobscot Drive
Redwood City, CA 94063(US)

(72) Inventor: Simpson, John B
116 Fox Hollow Road
Woodside California 94063(US)
Inventor: Gifford III, Hanson Smiley
712 Matadero Avenue
Palo Alto California 94306(US)
Inventor: Stenstrom, Kenneth A.
1348 Wawona Street
Manteca California 95336(US)

(74) Representative: Cross, Rupert Edward Blount
et al
BOULT, WADE & TENNANT 27 Furnival Street
London EC4A 1PO(GB)

## Description

This invention relates to a motor drive unit and more particularly to a motor drive unit for use with an atherectomy catheter device.

In EP-A-0163502 (Devices For Vascular Intervention) there is disclosed a motor drive unit for an atherectomy catheter device. However, it has been found that the motor device disclosed therein has certain disadvantages. For example, it is difficult to ascertain relative motion of a cutter driven by the unit with respect to the catheter device. In addition, it was necessary to hold the motor drive unit in one hand and the catheter device in the other hand to create relative motion between the two.

According to this invention there is provided a motor drive unit for use with an atherectomy device of the type having a flexible drive cable, the motor drive unit comprising a case, a motor mounted in the case, a power supply for the motor mounted in the case, switch means mounted in the case and accessible from the exterior of the case for energizing the motor from the power supply, co-operative engagement means driven by the motor and carried by the case and adapted to make connection with co-operative engagement means carried by the flexible drive cable, and finger operated means slidably mounted in the case for movement longitudinal of the case for moving the flexible drive cable in a direction longitudinally of its axis of rotation, whereby the flexible drive cable can be advanced and retracted in a direction longitudinal of its axis of rotation while it is being rotated by the motor characterised in that said co-operative engagement means carried by the case includes means for permitting said movement of the flexible drive cable longitudinally of its axis of rotation and in that said finger operated means engages the flexible drive cable and is slidably mounted in a slot provided in the case for said movement longitudinal of the case.

The motor drive unit of the invention has an inexpensive construction so that it can be disposed of after use.

This invention will now be described by way of example with reference to the drawing, in which:

Figure 1 is a side elevational view of a motor drive unit incorporating the present invention attached to an atherectomy device;

Figure 2 is an end elevational view looking along the line 2-2 of Figure 1;

Figure 3 is a cross-sectional view of the motor drive unit shown in Figure 1 taken along the line 3-3 of Figure 2;

Figure 4 is a cross-sectional view taken along the line 4-4 of Figure 3; and

Figure 5 is a cross-sectional view taken along the line 5-5 of Figure 3.

Referring to the drawing, the motor drive unit 11 comprises an elongate, substantially oval cross-section case 12 formed of any suitable material. For example, the case 12 can be formed of an injection molded plastics material so as to give the case an attractive appearance.

A gear head motor 13 is mounted in the case. The gear head motor 13 can be of a suitable type such as Micromo model 1624 with a 16/2 gear head. The gear head motor 13 is provided with an output shaft 14 which can be driven at suitable speed, as for example, from 1500 to 3000 rpm and preferably at a speed of approximately 2500 rpm. The gear head motor 13 is mounted in a compartment 16 in the case 12. A hollow shaft extension 19 is provided which has circumferentially spaced apart triangularly-shaped splines 20 therein on the interior surface thereof that serve as the cooperative connection means as hereinafter described. The outer extremity of the shaft extension 19 is rotatably mounted in a bearing 21 which is carried by the case 12. The bearing 21 as well as the hollow shaft extension 19 are disposed within a compartment 22 provided in the case 12.

A power supply is provided in the case and takes the form of a pair of batteries 26 which are mounted in a compartment 27 in the case immediately below the motor compartment 16. The batteries 26 are arranged in series and have one end of one battery engaging a coil spring 28 and having the other end of the other battery engaging a contact 29 carried by a switch 31. The switch 31 is mounted in a compartment 32 within the case and is provided with a threaded extension 33 which extends through a hole provided in the case 12. The extension 32 is held in place by a nut 36 threaded onto the extension. A finger operated push button 37 is carried by the extension 33 and is adapted to be engaged by a finger of the same hand which holds the case 12. Conducting wires 41 and 42 are provided for establishing an electrical connection between the batteries 26 and the gear head motor 13 under the control of the switch 31.

The case 12 is provided with a forwardly extending extension 46 which is provided with a tapered bore 47 facing forwardly therefrom. The bore 47 is in communication with a passage 48 provided in the extension and which opens into the hollow shaft extension 19.

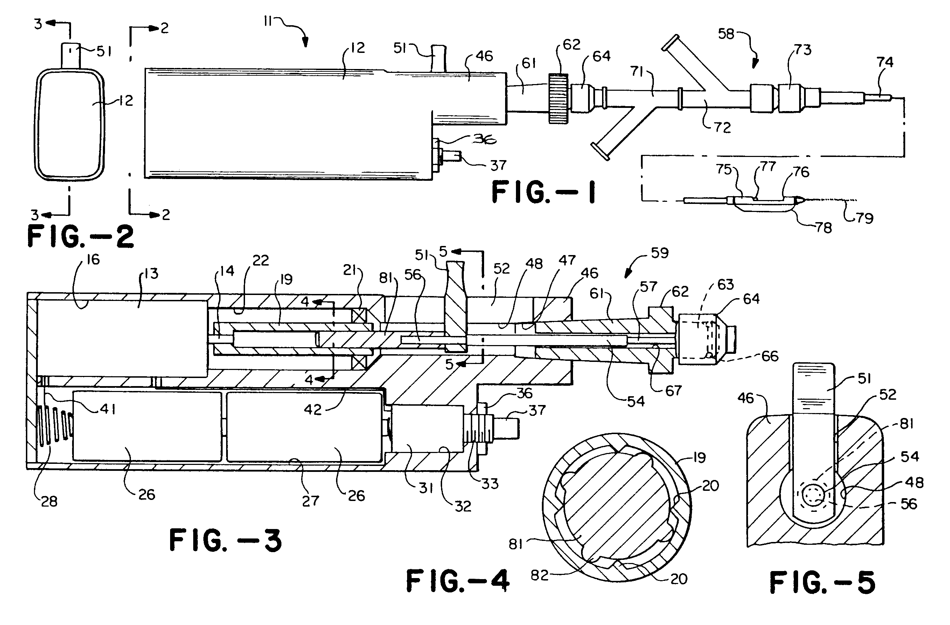

A finger operated slide or flipper member 51 is removably mounted in the extension 46 of the case 12 and extends through a slot 52 provided in the case 12. The lower extremity of the finger operated member 51 is disposed within the passage 48 and has a hole 54 (see Fig. 5) therein through which a shaft 56 rotatably extends. The shaft 56 is carried by the proximal extremity of the flexible drive cable 57 and is fastened thereto by suitable means such as crimping. The flexible drive cable 57 with the flipper member 51 carried thereby forms a part of an atherectomy device of the type described in EP-A- 0 163 502. As described therein, the atherectomy device 58 is comprised of a fitting 59 which is provided with a proximal tapered portion which is adapted to be seated within the tapered bore 47 of the extension 46 of the motor drive unit 11. The fitting 59 is provided with an knurled flange or knob 62 and with a threaded extension 63. The threaded extension 63 is adapted to be threaded into the proximal end of a fitting 64 and to form a fluid-tight seal with respect to an O-ring 66 carried by the fitting 64. The fitting 59 is provided with a bore 67 through which the shaft 56 extends.

The fitting 64 forms a part of the atherectomy device and as shown forms a part of an adapter 71. The adapter 71 is mounted in another adapter 72. The adapter 72 is mounted in a fitting 73 carried by the proximal extremity of a flexible guide tubing 74. A housing 75 having a cutout 76 is carried by the distal extremity of the flexible guide tubing 74. A cutter 77 is rotatably and slidably mounted in the housing 75 and is secured to the flexible drive cable 57. An inflatable balloon 78 is carried on the exterior of the housing on the side opposite where the cutout 77 appears. A flexible guide wire 79 is secured to the distal extremity of the housing 75.

A drive spline 81 is secured to the proximal extremity of the shaft 56 and is provided with circumferentially spaced apart elongate splines 82 which are semicircular in cross section. The drive spline 81 is sized so that it can readily enter the splined hollow drive shaft 19 by pushing the drive spline 81 into the shaft 19. The clearance between the spline 20 and 82 is such that an insertion can be made with ease. The finger member 51 is held in place by the drive spline 81 and is positioned so it can enter the slot 52 as the drive spline 81 is inserted into the splined hollow drive shaft 19. The drive spline 81 and the splined hollow drive shaft serve as cooperative connection means.

Operation and use of the motor drive unit 11 for operating an atherectomy device 58 will now be briefly described. Let it be assumed that the atherectomy device has been inserted into a vessel in the vascular system of a patient in the proper position and that the balloon 78 has been inflated and it is desired to operate the cutting device carried by the housing 75. To accomplish this, the proximal extremity of the atherectomy device 58 in the form of the drive spline 81 is inserted by introducing it into the passage 48 of the extension 46 of the case 12 and into the splined hollow shaft extension 19 carried by the case 12. At the same time the finger member 51 is positioned so it enters the slot 52 in the case 12. The drive spline 81 is held in place by urging the tapered portion 61 of the fitting 59 into the tapered bore 47 of the extension 46 of the case 12.

As soon as this has been accomplished, the case 12 can be grasped by the hand of the physician and the index finger of the hand can be utilized to operate the push button to supply energy to the drive motor 13 to cause operation of the same which in turn will cause rotation of the flexible drive cable 57 and the cutter 77 driven thereby at a relatively high speed as, for example, 2500 rpm. The thumb of the same hand of the physician can then be utilized for engaging the finger operated slide or finger member 51 to advance the finger member 51 in the slot 52. Movement of the finger member 51 moves the shaft 56 and the flexible drive cable 57 attached thereto and the cutter 77 attached to the drive cable 57. The slot 52 is of such a length so that the cutter carried by the housing 75 can be advanced throughout the entire length of the cutout 76 provided by the housing. After the finger member 51 has been moved as far forward as possible, it can be retracted by placing the thumb of the hand on the other side of the finger member 51 and pulling the same backwards in the slot 52.

Alternatively, the finger member 51 can be retained in its extreme forward position and the atherectomy device can be removed from the patient. The material which has been cut from the atheroma may be cleaned out of the housing and thereafter, the atherectomy device can be again inserted into the patient for a further cutting operation if that is still desired. In removing the atherectomy device, it is readily apparent that if desired, the motor drive unit 11 can be readily disconnected from the atherectomy device merely by separating the friction type fitting 61 from the housing and removing the drive spline 81 from the splined hollow shaft extension 19.

From the foregoing description it can be seen that the motor drive unit can be readily turned on and off by operating the switch 31. As explained previously, the switch 31 can be operated by the index finger of the hand which is holding the motor drive unit. Similarly, the thumb of the same hand holding the motor drive unit can be utilized for operating the finger member 51 for advancing and retracting the cutter in the housing of the atherectomy device. The position of the finger member 51 relative to the case 12 gives an excellent indication of the position of the cutter 77 in the cutout 76. The case is constructed in such a manner that the batteries and the motor drive can be inserted from the rear of the case. As can be seen, the device is constructed in such a manner that it can be manufactured relatively inexpensively so that if desired, the motor drive unit can be manufactured as a disposable device. If desired, the device can be reutilized by mere sterilization of the same. The construction of the motor drive unit is such that the atherectomy device can be readily connected to and disconnected from the motor drive unit.

## Claims

1. A motor drive unit (11) for use with an atherectomy device of the type having a flexible drive cable (57), the motor drive unit comprising a case (12), a motor (13) mounted in the case (12), a power supply (26) for the motor (13) mounted in the case (12), switch means (31) mounted in the case (12) and accessible from the exterior of the case (12) for energizing the motor (13) from the power supply (26), co-operative engagement means (19) driven by the motor (13) and carried by the case (12) and adapted to make connection with co-operative engagement means (81) carried by the flexible drive cable (57), and finger operated means (51) slidably mounted in the case (12) for movement longitudinal of the case for moving the flexible drive cable in a direction longitudinally of its axis of rotation, whereby the flexible drive cable (57) can be advanced and retracted in a direction longitudinal of its axis of rotation while it is being rotated by the motor (13) characterised in that said co-operative engagement means (19) carried by the case (12) includes means (20) for permitting said movement of the flexible drive cable (57) longitudinally of its axis of rotation and in that said finger operated means (51) engages the flexible drive cable (57) and is slidably mounted in a slot provided in the case (12) for said movement longitudinal of the case (12).

2. A motor drive unit as claimed in Claim 1, characterised in that the co-operative engagement means (19) carried by the case (12) and the co-operative engagement means (81) carried by the flexible drive cable (57) are in the form of splined connections.

3. A motor drive unit as claimed in Claims 1 or 2, characterised in that the finger operated means (51) has a portion which extends out of the case (12) in a direction which is substantially perpendicular to the axis of rotation of the co-operative engagement means (19, 81).

4. A motor drive unit as claimed in Claim 1, characterised in that the switch means (31) includes a push button (37) which is accessible at the forward extremity of the case (12).

## Patentansprüche

1. Motorantriebseinheit (11) für den Gebrauch mit einer Atherektomievorrichtung des Typs, der ein flexibles Antriebskabel (57) hat, wobei die Motorantriebseinheit
   - ein Gehäuse (12),
   - einen in dem Gehäuse (12) angebrachten Motor (13),
   - eine in dem Gehäuse (12) angebrachte Stromversorgung (26) für den Motor (13),
   - eine in dem Gehäuse (12) angebrachte und von außerhalb des Gehäuses (12) zugängliche Schalteinrichtung (31), um den Motor (13) von der Stromversorgung aus (26) zu aktivieren,
   - eine von dem Motor (13) angetriebene und von dem Gehäuse (12) getragene Zusammenwirkungseingriffseinrichtung (19), die zur Herstellung einer Verbindung mit einer von dem flexiblen Antriebskabel (57) getragenen Zusammenwirkungseingriffseinrichtung (81) geeignet ist, und
   - eine in dem Gehäuse (12) für eine Bewegung längs des Gehäuses verschiebbar angebrachte, fingerbetätigte Einrichtung (51) zum Bewegen des flexiblen Antriebskabels in einer Richtung längs seiner Drehachse aufweist, wodurch das flexible Antriebskabel (57) in einer Richtung längs seiner Drehachse vorgeschoben und zurückgezogen werden kann, während es durch den Motor (13) in Drehung versetzt wird,

   dadurch gekennzeichnet,
   - daß die von dem Gehäuse (12) getragene Zusammenwirkungseingriffseinrichtung (19) eine Einrichtung (20) aufweist, die die Bewegung des flexiblen Antriebskabels (57) längs seiner Drehachse ermöglicht, und
   - daß die fingerbetätigte Einrichtung (51) mit dem flexiblen Antriebskabel (57) in Eingriff steht und in einem in dem Gehäuse (12) für die Bewegung längs des Gehäuses (12) vorgesehenen Schlitz verschiebbar angebracht ist.

2. Motorantriebseinheit nach Anspruch 1, dadurch gekennzeichnet, daß die von dem Gehäuse (12) getragene Zusammenwirkungseingriffseinrichtung (19) und die von dem flexiblen Antriebskabel (57) getragene Zusammenwirkungseingriffseinrichtung (81) die Form von Keilnutverbindungen haben.

3. Motorantriebseinheit nach Anspruch 1 oder 2, dadurch gekennzeichnet, daß die fingerbetätigte Einrichtung (51) einen Abschnitt hat, der sich aus dem Gehäuse (12) heraus in einer Richtung erstreckt, die im wesentlichen senkrecht zu der Drehachse der Zusammenwirkungseingriffseinrichtungen (19, 81) ist.

4. Motorantriebseinheit nach Anspruch 1, dadurch gekennzeichnet, daß die Schalteinrichtung (31) einen Druckknopf (37) aufweist, der an dem vorderen Ende des Gehäuses (12) zugänglich ist.

## Revendications

1. Unité de commande motrice (11) utilisable avec un dispositif d'athérectomie du type comportant un câble de commande souple (57), l'unité de commande motrice comprenant un carter (12), un moteur (13) monté dans le carter (12), une alimentation (26) pour le moteur (13) montée dans le carter (12), un moyen de commutateur (31) monté dans le carter (12) et accessible de l'extérieur du carter (12) pour activer le moteur (13) à partir de l'alimentation (26), un moyen d'engagement coopérant (19) commandé par le moteur (13) et supporté par le carter (12) et adapté pour réaliser une connexion avec un moyen d'engagement coopérant (81) supporté par le câble de commande souple (57), et un moyen commandé par pression du doigt (51) monté de façon coulissable dans le carter (12) pour un déplacement longitudinal au carter afin de déplacer le câble de commande souple dans une direction longitudinale à son axe de rotation, de manière à pouvoir faire avancer et reculer le câble de commande souple (57) dans une direction longitudinale à son axe de rotation pendant qu'il est entraîné en rotation par le moteur (13), caractérisée en ce que le moyen d'engagement coopérant (19) supporté par le carter (12) comprend un moyen (20) pour permettre le déplacement précité du câble de commande souple (57) le long de son axe de rotation et en ce que le moyen commandé par pression du doigt (51) engage le câble de commande souple (57) et est monté de façon coulissable dans une encoche prévue dans le carter (12) pour ce déplacement longitudinal au carter (12).

2. Unité de commande motrice suivant la revendication 1, caractérisée en ce que le moyen d'engagement coopérant (19) supporté par le carter (12) et le moyen d'engagement coopérant (81) supporté par le câble d'engagement souple (57) sont sous la forme de connexions cannelées.

3. Unité de commande motrice suivant l'une ou l'autre des revendications 1 et 2, caractérisée en ce que le moyen commandé par pression du doigt (51) comporte une partie qui se prolonge hors du carter (12) dans une direction qui est sensiblement perpendiculaire à l'axe de rotation des moyens d'engagement coopérants (19, 81).

4. Unité de commande motrice suivant la revendication 1, caractérisée en ce que le moyen de commutateur (31) comprend un bouton-poussoir (37) qui est accessible à l'extrémité avant du carter (12).

FIG.—1

FIG.—2

FIG.—3

FIG.—4

FIG.—5

EP 0 244 058 B1